(19) Europäisches Patentamt / European Patent Office / Office européen des brevets

(11) **EP 3 279 662 B1**

(12) **EUROPEAN PATENT SPECIFICATION**

(45) Date of publication and mention of the grant of the patent:
**22.04.2020 Bulletin 2020/17**

(51) Int Cl.:
*G16B 25/30* (2019.01)   *G01N 33/558* (2006.01)

(21) Application number: **16001697.8**

(22) Date of filing: **01.08.2016**

(54) **LATERAL FLOW IMMUNOASSAY TECHNIQUE**

LATERAL-FLOW-IMMUNTESTASSAY

TECHNIQUE D'ESSAI BIOLOGIQUE À ÉCOULEMENT LATÉRAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**07.02.2018 Bulletin 2018/06**

(73) Proprietor: **Scienion AG**
**12489 Berlin (DE)**

(72) Inventors:
 • **Veen, Jeroen**
   **6861 ZT Oosterbeek (NL)**
 • **Arends, Hugo Antonie**
   **6851 NS Huissen (NL)**
 • **van Amerongen, Aart**
   **3906 ES Veenendaal (NL)**
 • **Koets, Maatje**
   **5612 ME Eindhoven (NL)**

(74) Representative: **v. Bezold & Partner Patentanwälte - PartG mbB**
   **Akademiestraße 7**
   **80799 München (DE)**

(56) References cited:
 • **D. J. CARTER ET AL: "Lateral flow microarrays: a novel platform for rapid nucleic acid detection based on miniaturized lateral flow chromatography", NUCLEIC ACIDS RESEARCH, vol. 35, no. 10, 22 April 2007 (2007-04-22), pages e74-e74, XP055008543, ISSN: 0305-1048, DOI: 10.1093/nar/gkm269**
 • **MARCIN CZAJKOWSKI ET AL: "Multi-test decision tree and its application to microarray data classification", ARTIFICIAL INTELLIGENCE IN MEDICINE, vol. 61, no. 1, 1 May 2014 (2014-05-01), pages 35-44, XP055340123, NL ISSN: 0933-3657, DOI: 10.1016/j.artmed.2014.01.005**
 • **RICHA TIWARI: "Comparison of microarray image analysis software", PROCEEDING ACM-SE 46 PROCEEDINGS OF THE 46TH ANNUAL SOUTHEAST REGIONAL CONFERENCE ON XX, 1 January 2008 (2008-01-01), page 77, XP055383626, ACM New York, NY, USA DOI: 10.1145/1593105.1593125 ISBN: 978-1-60558-105-7**

## Description

### Technical Field

The present disclosure generally relates to a technique for performing an immunoassay of analytes in a sample. More specifically, and without limitation, the disclosure provides a device and a method for evaluating the immunoassay during lateral flow.

### Background

Immunoassays allow detecting analyte molecules (or short: analytes) in a sample by means of binding molecules. Immunoassays can detect a panel of analytes encompassing proteins, microorganisms, DNA amplicons, RNA amplicons, toxins and antibiotics. For example, the presence of the analytes may be detected using a binding reaction between an antigen and an antibody, either one of which may take the role of the analyte and the binding molecule, respectively.

The lateral flow of an assay buffer carries the analytes along a membrane. Transversal stripes on the membrane include stationary binding molecules. Conventional immunoassays, such as a pregnancy hormone test, are a one-step assay to measure hormones in a sample. That is, the assay is optically (e.g., visually) evaluated when the lateral flow has terminated in an equilibrium state of the assay buffer.

The article "Lateral flow microarrays: a novel platform for rapid nucleic acid detection based on miniaturized lateral flow chromatography", D. J. Carter and R. B. Cary, Nucleic Acids Research, 2007, Vol. 32, No. 10, e74, describes a system for field deployable nucleic acid detection. The system comprises a microarray platform and a miniaturized lateral flow device using hybridization-mediated target capture. Ten-microliter sample volumes reduce reagent consumption and yield analyte detection times of less than 120 s, while providing sub-femtomole sensitivity. Moreover, the use of microarray technology increases the potential information capacity of lateral flow. The lateral flow microarray system is demonstrated using an isothermal amplification strategy for detection of Bacillus anthracis, the etiologic agent of anthrax. RNA from as few as two B. anthracis cells was detected without thermocycling hardware or fluorescence detection systems.

More complex applications use a Lateral Flow Microarray Immunoassay (LMIA) or, if the LMIA is used for detecting DNA amplicons, Nucleic Acid LMIA (NALMIA). As the complexity of the analytes in the sample increases, the immunoassay has to be performed in conjunction with multiple types of binding molecules. However, performing, sequentially or in parallel, a conventional immunoassay for multiple types of binding molecules would require awaiting the equilibrium state for each of the binding molecules.

## Summary

Accordingly, there is a need for a technique that allows performing an immunoassay more rapidly.

As to one aspect, a device for performing an immunoassay of analytes in a sample according to claim 1 is provided. The device comprises a membrane including a first site, a second site laterally separated from the first site and multiple spots arranged between the first site and the second site, each spot including binding molecules for selectively binding the analytes at the corresponding spot; a liquid phase configured to carry the sample and labels for optically signaling the presence of the analytes, and to laterally flow from the first site across the spots to the second site; and an image acquisition unit configured to acquire a sequence of images, wherein each image captures the multiple spots, and the sequence of images is indicative of optical signals from the labels at different points in time during the lateral flow.

By acquiring a temporal sequence of images, each image being indicative of the optical signals from the multiple spots that include the binding molecules for selectively binding the analytes, additional information as to the temporal evolution of binding reactions at the multiple spots can be acquired. The additional information of developing spots may allow detecting or quantifying the analytes more rapidly, e.g., even before the lateral flow terminates, the liquid phase evaporates or asymptotically approaches an equilibrium state. Alternatively or in addition, the additional information may allow detecting or quantifying the analytes more accurately.

By including multiple spots in the lateral flow along the membrane, a statistical significance of the optical signals captured in the images can be improved and/or more complex immunoassays may be performed. In a first embodiment, a hydrodynamic correlation between signals from different spots is compensated using the additional temporal information, e.g. for increasing the sensitivity of the immunoassay without losing specificity as the number of spots is increased. The compensation may be automated, e.g., by including redundant spots and/or reference spots on the membrane and computing weighting factors based on the optical signals from the redundant spots and/or the reference spots.

The analytes may also be referred to as analyte molecules. The plural of "analytes" may refer to a plurality of chemically or biologically different analyte molecules, rather than a plural in terms of the number of molecules in the sample.

The binding molecules may be localized at the spots. The liquid phase may wet the multiple spots. The binding molecules may be immobile under the lateral flow of the liquid phase.

Selectively binding may encompass that, at each spot, only one of the analytes or a specific group of the analytes is bound. The binding molecules may include antibody molecules. Each spot may include a specific set of antibody molecules. The analytes may include antigens.

The term "immunoassay", as used herein, may encompasses assays using antibodies without being restricted thereto. For example, non-immune ligands may be used as well (e.g., aptamers, oligo's, etc.) in the "immunoassay". Moreover, the "immunoassay" may also encompass an enzymatic assay. A substrate in the running buffer (e.g., in the liquid phase) may be converted to a precipitable dye by a spot-immobilized enzyme.

The liquid phase may enter the membrane at the first site. The liquid phase may be injected onto the membrane at the first site or onto a pad (e.g., a buffer pad or a sample pad) directly or indirectly (e.g., via a conjugate pad) in contact with the first site. The liquid phase may exit the membrane at the second site. The liquid phase may be collected from the membrane at the second site and/or by a pad (e.g., an absorbance pad) directly or indirectly in contact with the second site. The membrane may be a nitrocellulose membrane.

The liquid phase may include an assay buffer, e.g., for defining a pH value. Alternatively or in addition, the liquid phase may include surfactants or other additives such as an excess of an inert protein to block the membrane during the flow.

The image acquisition unit may be configured to laterally resolve the multiple spots in each image of the sequence.

The device may further include a source (e.g., a light source) configured to irradiate electromagnetic radiation (e.g., light) on the multiple spots. The irradiated light may induce the optical signal from the labels. The optical signal may include at least one of reflected light and luminescent (e.g., fluorescence) light.

The device further comprises a controller coupled to the image acquisition unit. The controller is configured to analyze the sequence of images during the lateral flow. Optionally, the controller may be configured to output a result of the immunoassay based on the sequence of images, e.g., during the lateral flow. The analysis may include calculations based on the acquired images. The output may be a result of the calculations. The result of the immunoassay may be output on a display coupled to the controller.

The controller may analyze the sequence in real-time. The sequence of images may be referred to as a real-time sequence. Alternatively or in addition, the controller may output the result in real-time. For example, the controller may repeat and/or update the analysis and/or the output (e.g., every time) as a further image of the sequence is acquired. The images may be analyzed and/or the result may be output on the basis of the acquisition of the sequence of images from the start of the acquisition and/or as soon as a sufficient number of images or as soon as significant images have been acquired to perform the analysis and/or calculate the result.

The controller may be configured to segment each image of the sequence into subimages. Each subimage may include only one of the spots. Each subimage may include a different one of the spots.

The controller may be configured to retrieve coordinates for the multiple spots or the subimages from memory, e.g., at the device. Alternatively or in addition, the controller may determine coordinates for the multiple spots or the subimages by processing at least one of the images. The coordinates may be defined relative to the images as acquired by the unit or relative to markers, e.g., on the membrane. The stored coordinates may define initial values for an image recognition determining the multiple spots or subimages.

The same coordinates may be applied to each of the images in the sequence.

The coordinates may be determined by processing each of the images in the sequence and/or by processing an initial image of the spots. The spots may include an inert dye. The coordinates may be determined using pixels in the one or more images representing the inert dye. This is also referred to as spatial function of the inert dye. Alternatively or in addition, the membrane may include markers (e.g., markers for position and/or orientation of the membrane and/or the multiple spots). The coordinates may be determined using pixels in the one or more images representing the markers.

The subimages may be defined by Delaunay triangulation of the coordinates. The coordinates may correspond to centers of the spots. The subimages may also be referred to as regions of interest (ROI).

Each of the subimages may include a foreground and a background of the corresponding one spot. The foreground may include pixels corresponding to an area of the binding molecules of the spot. The background may include pixels corresponding to an environment of the spot, e.g., outside of the area of the binding molecules.

The controller may be configured to separate the foreground and the background. The foreground and the background may be separated based on at least one of intensity, color, contrast, contour and texture.

The coordinates may be determined based on the foreground, e.g., by computing a centroid of the foreground. The foreground may be (e.g., by definition) the complement to the background within the subimage. Alternatively or in addition, the background may be (e.g., by definition) the complement to the foreground within the subimage.

Alternatively or in addition, the coordinates and/or the foreground may be determined using pattern recognition. The pattern recognition may include cross-correlating a (e.g., stored) profile of a template spot (e.g., a profile in terms of intensity, color, and/or contour) with the acquired image.

The controller is further configured to extract signal features of each of the multiple spots for each of the points in time of the sequence. For example, the extracted signal features may be stored and/or structured as a function of the points in time and the multiple spots (e.g., using a matrix indexed by time and spot).

The signal features may be extracted for each of the foreground and the background of the corresponding spot. Each spot may be associated with a set of signal features. Each set may include a first subset of signal features extracted from the foreground of the corresponding spot and a second subset of signal features extracted from the background of the corresponding spot.

The signal features may represent statistics (e.g., moments, cumulants, an average and/or a variance) of pixels in the corresponding spot or in corresponding subimage. The statistics may be based on intensity and/or color of the pixels. The signal features may be computed as a function of moments or cumulants (e.g., of the intensity or the color) of the foreground and/or the background of the corresponding spot.

Alternatively or in addition, the signal features may include at least one of a mean intensity of the spot, a variance of the intensity, a contrast of the spot, a texture of the spot, a contour of the spot, shape of the spot and an area size of the spot.

The controller is further configured to determine parameters of at least one signal model for each of the multiple spots based on the extracted signal features of the corresponding spot. One or more parameters of at least one of the multiple spots may be determined using one or more signal models (also: spot-specific models) that are uniquely used for one of the multiple spots.

The signal models may include statistical models or estimators. The parameters may represent statistics (e.g., moments, cumulants, an average and/or a variance) of one or more of the signal features (e.g., correlations between different signal features).

Alternatively or in addition, the signal models may be time-dependent. Determining the parameters may encompass estimating the parameters or extrapolating a trend (e.g., beyond the currently acquired points in time), e.g., using the signal model to represent the extracted signal features, optionally as a function of the points in time. The parameters may be determined based on a (e.g., linear) regression of the signal model, a moving average (e.g., moving along the points in time) and/or exponential smoothing (e.g., double-exponential smoothing) of one or more of the extracted signal features.

At least one or each of the signal models may include a dependency on the points in time of the sequence. The controller may determine the parameters of each spot based on one or more of the signal features of the corresponding spot for the points in time of the sequence, e.g., acquired up until real-time and/or during the lateral flow.

The signal models may represent a trend, a slope, a base line and/or an asymptote of one or more of the signal features as a function of the points in time. For example, the analysis may include computing the slope of the intensity or the color of pixels in a certain image region (e.g., the subimage of a particular spot) over the sequence of images.

The controller may be configured to determine and/or indicate completion of the acquisition and/or the analysis, e.g., depending on a goodness of the determined parameters. At least one of the acquisition, the analysis and the immunoassay may be completed prior to the parameters reaching a plateau. Completion may be indicated during a slope of the parameters (or a slope of the underlying signal features) as a function of the points in time. Indicating completion may include outputting the result or a final result (e.g., a quantitative result).

The controller is further configured to compare the parameters with one or more threshold values. Optionally, the controller may be configured to output the result of the immunoassay based on the comparison.

The comparison may be performed for each of the points in time and/or for each of the multiple spots. At least some of the threshold values may be different for different spots.

The controller may be configured to generate a binary matrix based on the comparison. The binary matrix may be indicative of the result of the comparison for each of the multiple spots, e.g., in conjunction with each of the points in time.

The binary matrix may be input to a binary decision tree. The result of the immunoassay may be based on one or more outputs of the binary decision tree. A number of binary inputs to the binary decision tree may be greater than (e.g., multiple times greater than) a number of binary outputs from the binary decision tree.

At least one or all of the threshold values may be stored at the device. Alternatively or in addition, the controller may compute the threshold values according to a threshold model, e.g., depending on at least one of the parameters. The threshold values may depend on a variance of one or more of the parameters (e.g., according to the threshold model). The threshold value for one of the parameters may depend on the variance of another one of the parameters. The threshold model may not include an explicit time-dependency.

The controller may be configured to estimate the quantity or concentration of one or more of the analytes, e.g., based on a subset of the parameters. The subset may be defined by those parameters that exceed their threshold values.

The analysis may include at least one of segmenting each of the images, separating the background and the foreground, extracting the signal features, determining the model parameters, computing the threshold values, comparing the parameters, generating the binary matrix, parsing the binary decision tree and outputting the result.

The spots may be arranged in a lattice structure (e.g., a hexagonal lattice) or an array. The array may include two or more rows of spots. Each row may extend between the first site and the second site. The array may be a rectangular array.

Alternatively or in addition, some or all spots may be arranged in a zig-zag formation or a meander formation. For example, the spots may be arranged in rows that are arranged in a zig-zag formation so that spots in a more downstream row are not positioned in the wake of the spots of the preceding row.

The threshold values (e.g., as computed using the threshold model) may be shifted by (e.g., adding or subtracting) offset values assigned to each of the spots. In a second embodiment, which may be combinable with the first embodiment, the threshold values are scaled (e.g., multiplied) by weighting factors assigned to each of the spots. At least one of the offsets values and the weighting factors in at least one of the rows may decrease from the spot closest to the first site to the spot closest to the second site.

In the first embodiment, spots that are in the direction of the lateral flow downstream of other spots receive less analytes than the other spots. Assigning correspondingly lower threshold values to downstream spots compensates such a geometrically caused gradient in the concentration of one or more of the analytes.

For example, the array may include two or more identical spots on different columns (perpendicular to rows in the direction of the lateral flow) of the array. The at least one spot on the most downstream column may receive less analyte molecules, e.g., since spots at the more upstream columns have captured analytes molecules out of the flow of the liquid phase. The identical spots may be used in the analysis to improve statistical significance (e.g., reducing a variance of the extracted signal features or the determined model parameters) and/or for verifying the result. "Identical spots" may encompass spots that include an identical amount or density of binding molecules for at least one of the analytes. Three identical spots may also be referred to as a "triplo".

In the second embodiment, different weighting factors are assigned to compensate different flow rates or different velocities of the liquid phase. As one example, the flow rate and/or the flow velocity may decrease (e.g., according to the continuity equation) due to evaporation of the liquid from the membrane, e.g., in the first half of the membrane. As a second example (combinable with the first example), the flow rate and/or the flow velocity of the liquid phase may increase towards the second site and/or an absorbance pad. The flow rate or the flow velocity may influence the binding kinetics between the analyte molecules and the binding molecules (optionally, also depending on the particular phase in the run time of the immunoassay).

Based on a time difference or time lag between signal features or model parameters of the two or more identical spots, or two or more control spots that change color and/or intensity independent of the analytes, the controller may estimate the flow rate and/or the flow velocity of the liquid phase at the membrane. Alternatively or in addition, the controller may use signal features or model parameters (e.g., as a function of the points in time) of the identical spots or the control spots as a time-dependent reference (e.g., as a time-dependent threshold value) for analyzing the signal features or model parameters from other spots.

The weighting factors may depend on the time difference or the time-sequence of the signal features or model parameters retrieved from the identical spots or the control spots. Alternatively or in addition, the weighting factors may depend on the time difference or the time-sequence of the initial signals (e.g., intensity or color) of spots including the inert dye that is dissolved in the liquid phase.

The control spots may be used as a reference spot. Alternatively or in addition, the control spots may be created by adding an independent (e.g., second or further) analyte to the liquid phase. Optionally, the control spots may be created by adding an independent analyte with its own (e.g., separate) nanoparticles conjugate (e.g., that are not influenced by a decreasing concentration of the conjugate of the one or more primary analytes). Alternatively or in addition, one or more of the control spots may include an inert colored material (e.g., added during printing the spots) that is immobile (e.g., stays bound to the membrane) irrespective of the flow of the liquid phase across the spots.

Alternatively or in addition, the weighting factors may compensate for signal intensities of the spot that depend (e.g., in the direction of the lateral flow) on the concentration of analytes and reagents, such as the concentration of colored nanoparticles that are used in the immunoassay to specifically stain spots with bound analytes.

The weighting factors may be determined depending on at least one of the type of pads, the type of membrane, concentrations of reagents, location of the corresponding spots, external conditions (e.g., evaporation rate, temperature). For a given combination or subcombination of above of conditions (e.g., types of pads and type of membrane), the device may be configured to perform a calibration run. Alternatively or in addition, the controller may retrieve the weighting factors from standard curves for the analytes stored at the device. Alternatively or in addition, the controller may be configured to statistically analyze a pattern of the spots in the images per analyte concentration or retrieve a stored statistical analysis.

Each of the multiple spots may further include an inert dye. The inert dye may cause an initial slope (e.g., a negative slope) in the optical signal (e.g., the color or the intensity). The initial slope may define a starting point for the points of time of the sequence. This is also referred to as temporal function of the inert dye.

The multiple spots may be different in at least one of a concentration of the binding molecules and a selectivity of the binding molecules. The controller may be configured to determine a concentration of at least one of the analytes by fitting the parameters of spots including different concentrations of the same binding molecules (e.g., selective to the analyte) to a sensitivity curve of said binding molecules. The sensitivity curve may be stored at the device.

The membrane may be configured to enable or induce the lateral flow of the liquid phase, e.g., by at least one of capillary action and gravity. A pad may be arranged at the second site of the membrane for collecting the liquid phase.

The membrane may further include or contact a source for the labels. The source may be a conjugate pad. The source may contact the first site or may be arranged at the first site or between the first site and the multiple spots.

As to another aspect, a method of performing an immunoassay of analytes in a sample according to claim 21 is provided. The method comprises a step of providing a membrane including a first site, a second site laterally separated from the first site and multiple spots arranged between the first site and the second site, each spot including binding molecules for selectively binding the analytes at the corresponding spot; a step of providing a liquid phase configured to carry the sample and labels for optically signaling the presence of the analytes, and to laterally flow from the first site across the spots to the second site; and a step of acquiring a sequence of images, wherein each image captures the multiple spots, and the sequence of images is indicative of optical signals from the labels at different points in time during the lateral flow.

The method may further include any step, or a corresponding step, disclosed in the context of the device aspect. For example, the method may include a step of providing any feature disclosed for the device aspect.

As to a further aspect, a computer program product is provided. The computer program product comprises program code portions for performing any one of the steps of the method aspects disclosed herein (e.g., the analysis) when the computer program product is executed by one or more computing devices. The program code portions may be executed by the controller, by another unit of the device or at a local computer.

Alternatively or in addition, program code portions may be executed remotely on a server communicating with the device, e.g., with the controller. E.g., a first portion of the program code may be performed by the controller. The controller may send the acquired sequence of images (e.g., image by image as they are acquired) to the server and/or may receive the result of analyzing the current sequence or the final result of the immunoassay from the server (e.g., for local output on the display). A second portion of the program code may be performed by the server. Particularly, one or more steps related to the analysis may be executed by the server.

As to a variant of the product aspect, a first computer program product comprising the first portion of the program code is provided and/or a second computer program product comprising the second portion of the program code is provided.

The computer program product or each of the computer program products may be stored on a computer-readable recording medium. The computer program product or each of the computer program products may also be provided for download via a data network, e.g., the Internet.

Furthermore, any data stored at the device may be downloaded or updated by means of the data network.

**Brief Description of the Drawings**

Further details of embodiments of the technique are described with reference to the enclosed drawings, wherein:

Fig. 1 schematically illustrates an embodiment of a device for performing an immunoassay;

Fig. 2 shows a flowchart of a first implementation of a method of evaluating an immunoassay, e.g., the immunoassay performed by the device of Fig. 1;

Fig. 3 shows a flowchart of a second implementation of a method of evaluating an immunoassay, e.g., the immunoassay performed by the device of Fig. 1;

Fig. 4 shows a flowchart of an implementation of a step of extracting signal features;

Fig. 5 shows a flowchart of an implementation of a step of determining model parameters;

Fig. 6 schematically illustrates a time-dependent signal feature, e.g., extracted according to Fig. 4 and analyzed according to Fig. 5; and

Fig. 7 shows a flowchart of an implementation of a step of outputting a result of the immunoassay based on a comparison of the parameters.

**Detailed Description**

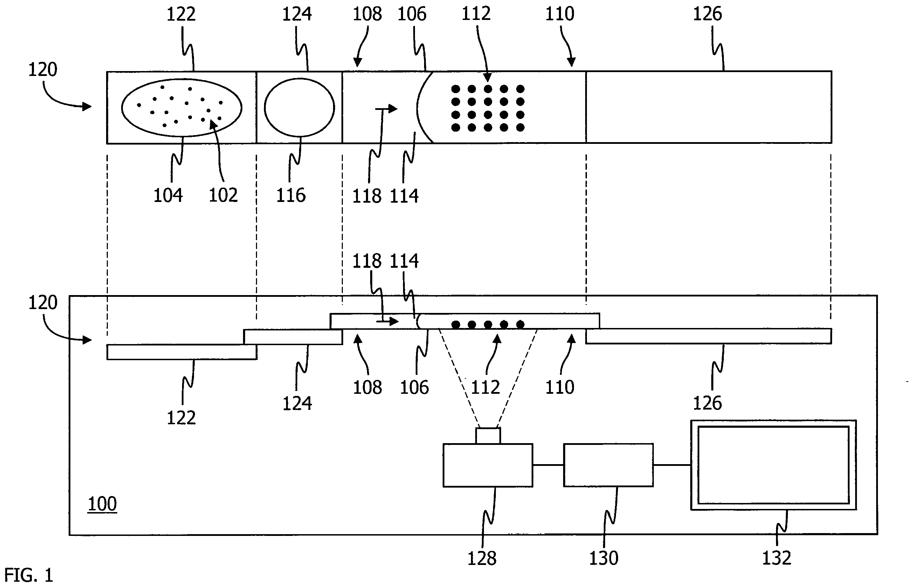

Fig. 1 schematically illustrates an embodiment of a device 100 for performing an immunoassay. The immunoassay detects and/or quantifies analytes 102 in a sample 104.

The device 100 comprises a membrane 106 or is configured to receive the membrane 106. The membrane 106 includes a first site 108, a second site 110 that is laterally separated from the first site 108. Multiple spots 112 are arranged in or on the membrane 106 between the first site 108 and the second site 110. Each of the spots 112 includes binding molecules for selectively binding the analytes 102 at the corresponding spot 112. The selectivity of the binding molecules means that different analytes 102 are trapped at different spots 112 including different binding molecules.

The device 100 further comprises a liquid phase 114. For example, the device 100 comprises a reservoir holding the liquid phase 114. In Fig. 1, the liquid phase 114 is schematically indicated by its phase boundary (curved line in Fig. 1). In the instance shown in Fig. 1, the liquid phase 114 is to the left of the phase boundary.

The liquid phase 114 is configured to carry the sample 104 and labels 116 for optically signaling the presence of the analytes 102 in a lateral flow 118 from the first site 108 across the spots 112 to the second site 110. The phase boundary is absent in a steady state of the lateral flow.

The spots 112 may initially include an inert dye that is washed out as the phase boundary traverses the spots 112. Alternatively or in addition to above-mentioned temporal and spatial functions of the inert dye, the inert dye may be incorporated in the spots 112 for controlling quality of a printing process that prints the spots on the membrane 106 and/or as markers that control a cutting device that cuts a larger membrane to the membrane 106 by optically detecting the spots.

The membrane 106 may include nitrocellulose. The membrane 106 may be included in a lateral flow strip 120. The liquid phase 114 may be driven by capillary or gravity through the lateral flow strip 120.

The lateral flow strip 120 may include (in the direction of the lateral flow 118) a sample pad 122 receiving the sample 102, a conjugate pad 124 including a reservoir of the labels 116, the membrane 106 and an absorbance pad 126 causing the capillary force in the steady state of the lateral flow 118. The conjugate pad 124 may contact the first site 108 of the membrane 106. The absorbance pad 126 may contact the second site 110 of the membrane 106.

The device 100 further comprises an image acquisition unit 128 configured to acquire a sequence of images. Each image captures the multiple spots 112 on the membrane 106. The device 100 may further comprise a light source arranged for irradiating light on the membrane 106 (e.g., in reflection or in transmission).

While the side view of the lateral flow strip 120 in Fig. 1 shows the membrane 106 with a distal offset from the image acquisition unit 128, the membrane 106 may also be offset towards the image acquisition unit 128, e.g., by interchanging the faces at the contacting overlaps between pads and the membrane 106. Alternatively or in addition, the contacting overlaps may include a kink or a step so that the pads and the membrane 106 are in one plane.

A lateral resolution of each image is multiple times greater than an average diameter of the spots 112, so that each spot 112 is represented by a plurality of pixels. Each pixel is indicative of intensity and/or color of the optical signal emitted from the membrane 110 at the corresponding lateral location. A sensitivity of the image acquisition unit 128 for one or more colors may be matched with an emission wavelength of the labels 116. The color is not necessarily in the visual spectrum and may include the ultraviolet (UV) spectrum and/or the (e.g., near) infrared (IR) spectrum.

In another embodiment, which may be combined with the embodiment of Fig. 1, the image acquisition unit 128 is configured to acquire the images by scanning local electrochemical states and/or local magnetic states of the membrane 106.

The sequence of images is acquired during the lateral flow 118, so that the images are indicative of optical signals emitted by the labels 116 at different points in time. A controller 130 of the device 100 is coupled to the image acquisition unit 128, analyzes the sequence of images during the lateral flow 118 and optionally outputs a result of the analysis on a display 132 (which may be internal or external to the device 100). The sequence of images may be represented or stored as a 4-dimensional array according to 3 colors and the points in time.

The controller 130 optionally includes a network interface for communicating via a data network, e.g., with a server.

The controller 130 extracts signal features of each of the multiple spots 112 for each of the points in time of the sequence, determines parameters of at least one signal model for each of the multiple spots based on the extracted signal features of the corresponding spot and compares the parameters with one or more threshold values.

In a first embodiment, a hydrodynamic correlation between signals from different spots 112 is compensated using the temporal sequence of images, the hydrodynamic correlation comprising spots 112 in the direction of the lateral flow 118 downstream of other spots 112 receiving less analytes than the other spots 112. The controller 130 assigns correspondingly lower threshold values to downstream spots 112 to compensate such a geometrically caused gradient in the concentration of one or more of the analytes.

In a second embodiment, which may be combinable with the first embodiment, the controller 130 scales the threshold values by weighting factors assigned to each of the spots 112. The different weighting factors are assigned to compensate different flow rates or different velocities of the liquid phase 114.

A method 200 of analyzing (or evaluating) an immunoassay includes a step 202 of acquiring a further image 134 (e.g., an N-th image), a step 204 of analyzing the immunoassay based on the acquired sequence of images 134 (e.g., numbered n=1..N), and a step 206 of outputting the result of the immunoassay based on the analysis 204. The index n for the images 134 in the sequence indicates the corresponding point in time when the image 134 was captured. The analysis 204 is performed as more and more images 134 are acquired during the lateral flow 118. The result is output (e.g., as a preliminary estimate) during the image acquisition 202 or (e.g., as a final result) as soon as reliability or accuracy of the analysis 204 is sufficient.

The method 200 may be performed by the device 100. For example, the image acquisition unit 128, the controller 130 and the display 132 may perform the steps 202, 204 and 206, respectively.

In a first implementation of the method 200, a sequence of features of the optical signals is maintained during the lateral flow, and the analysis 204 is based on the maintained sequence of features. In a second implementation of the method 200 (which is combinable with the first implementation), the sequence of features is derived from the sequence of images 134 every time a further image 134 is acquired. The second implementation allows analyzing each of the images 134 in the sequence depending on the further image 134.

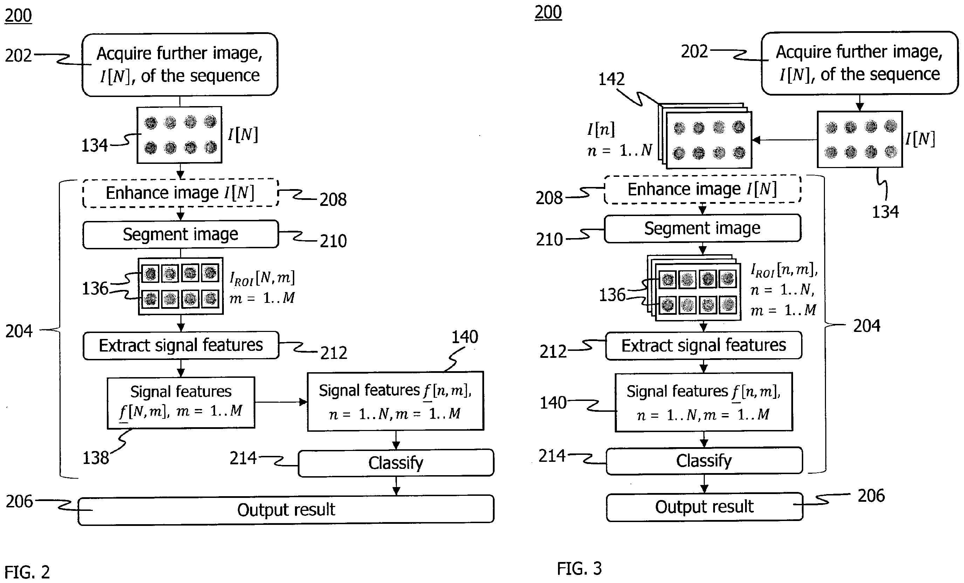

Fig. 2 shows an example flowchart of the first implementation of the method 200. The analysis 204 optionally includes a step 208 of enhancing or filtering each of the images 134. The step 208 may process each image 134 without distinguishing between the spots 112 included therein. The step 208 may include a white balance or adjusting contrast of the image 134.

The analysis 204 includes a step 210 of segmenting each image 134 in regions of interest (ROI) or subimages 136. Each subimage includes another one of the spots 112. The subimages 136 (and, thus, the spots 112) are numbered by $m=1..M$.

Signal features 138 for each of the $M$ spots 112 are extracted from the further $N$-th image 134 in a step 212. The extracted further signal features 138 are included in a sequence 140 of signal features. The sequence 140 of signal features includes, for each combination of image number $n$ (or point in time) and spot number $m$, a list of extracted signal features, $\underline{f}[n,m]$.

The sequence 140 of signal features is classified in a step 214. The step 214 may include determining parameters of a signal model based on the signal features, comparing the determined parameters with threshold values and logically combining the result of the comparison.

Fig. 3 shows an example flowchart of the second implementation of the method 200. The further image 134 is included in the sequence 142 of images stored by the device 100. The analysis 204 is performed for each image 134 in the sequence 142.

Features and steps indicated by like reference numbers correspond to those of Fig. 3.

The analysis 204 optionally includes a step 208 of enhancing or filtering each of the images 134. In the step 208, the image filter may be controlled once based on the sequence 142 of images 134 and equally applied to each image 134 in the sequence 142.

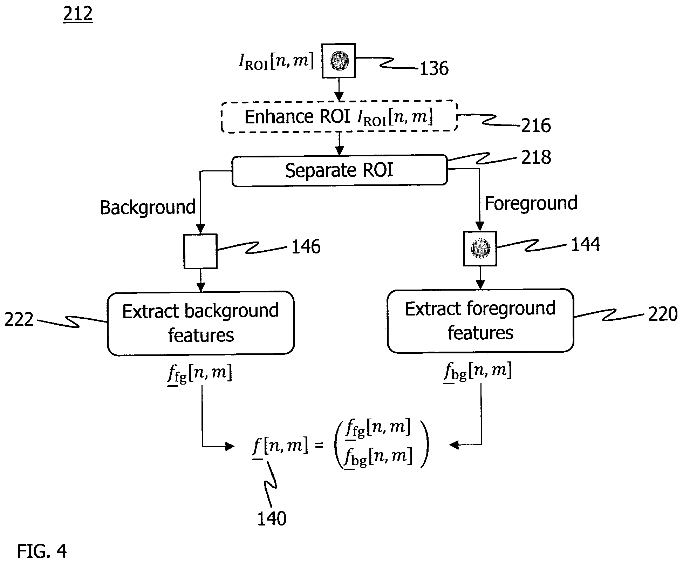

Fig. 4 shows a flowchart of an implementation of the step 212 of extracting signal features. The extracting 212 optionally includes a step 216 of enhancing or filtering the subimage 136 specifically for the corresponding $m$-th spot and the n-th point in time.

The subimage 136 is separated into a foreground 144 of the spot 112 and a background 146 in a step 218. Signal features are extracted from the foreground 144 and from the background 146 in steps 220 and 222, respectively. The foreground features and the background features are stored in association as the signal features 140 of the $m$-th spot at the $n$-th point in time. By way of example, signal features 140 include

$$\underline{f}[n,m] = \begin{Bmatrix} \mu_f, \sigma_f, I_f, A_f, ... \\ \mu_b, \sigma_b, I_b, A_b, ... \end{Bmatrix}.$$

Herein, $\mu_f$ and $\sigma_f$ are the mean and the variance, respectively, (e.g., of color) for the foreground 144 of the $m$-th spot at the $n$-th point in time. $I_f$ and $A_f$ are the (mean) intensity and area size, respectively, for the foreground 144 of the $m$-th spot at the $n$-th point in time. Corresponding signal features with lower index "b" are extracted from the background 146.

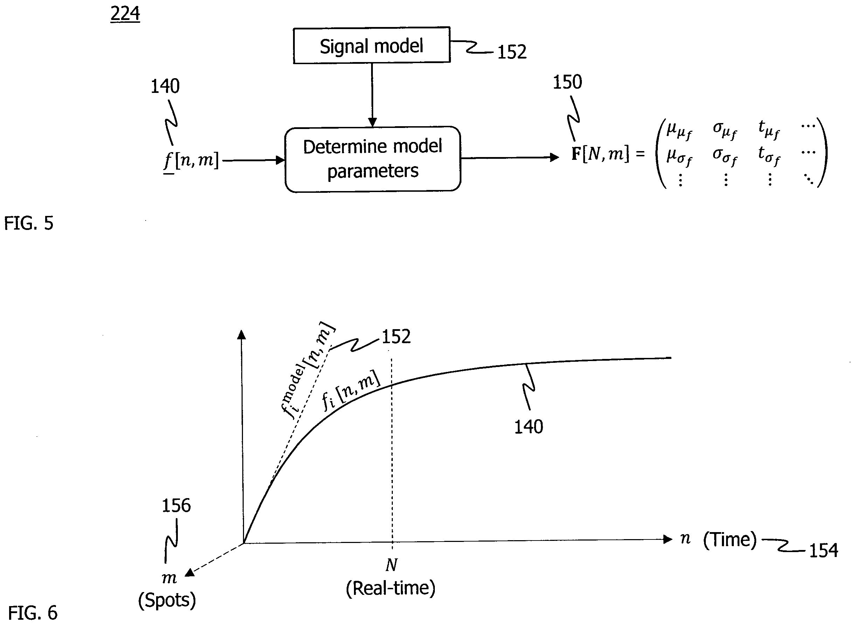

Fig. 5 shows a flowchart of an implementation of the step 224 of determining model parameters. For example, the change of one or more of the signal features during the lateral flow, for time $n=1..N,$ are represented or approximated by means of a signal model 152. The signal model 152 includes model parameters 150, which are determined so that the parameterized signal model reproduces or approximates the measured one or more signal features.

The step 224 is performed for each spot $m$. Since the step 224 is repeated as further images 134 are acquired, the parameters 150 are also a function of time $N$.

Fig. 6 schematically illustrates one of the time-dependent signal features 140. The signal model 152 for the corresponding one of the signal features 140 is fitted over a time axis 154, i.e., for the points in time $n=1..N$ up until real-time $N$.

By way of example, a signal model 152 for the intensity $I$ may include

$$f_i[n,m] = a + t \cdot n + \epsilon,$$

wherein the parameter $a$ is an offset value, the parameter $t$ is a slope, and $\varepsilon$ is an error term.

The error term is an example for determining the goodness of the determined parameters. Based on the value of the error term, the controller 130 determines when the analysis 204 is completed.

One or more of the signal models 152 may represent one or more of the signal features 140 as a function of time 154. Optionally, one or more of the signal model 152 represent a subset including two or more of the spots 112. The corresponding model parameters are determined by fitting the signal model also over the spots $m$ in the subset (which is indicated by the further axis 156).

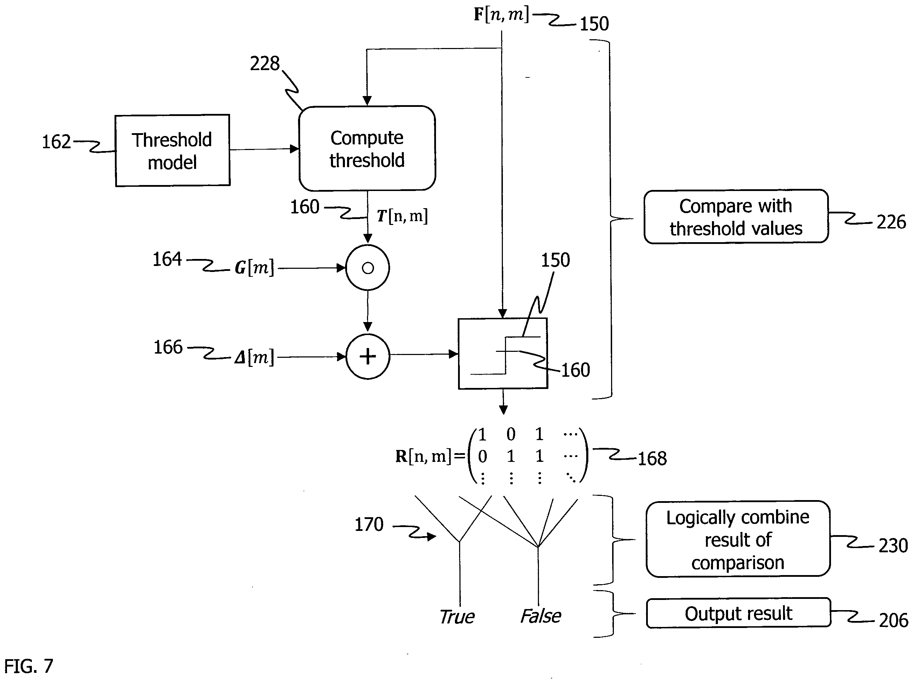

Fig. 7 shows a flowchart of an implementation of a step 226 of comparing the model parameters 150 and a step 230 of deriving the result of the immunoassay based on the comparison 226. The classifying step 214 may include the steps 226 and 230.

The parameters may be compared with threshold values 160. Optionally, the threshold values 160 are computed based on the parameters 150 in a step 228. For example, the threshold values are adaptive to statistics of the parameters 150.

For example, the threshold values 160 are computed according to a threshold model 162. The threshold model 162 may represent prior knowledge, e.g., a correlation between a parameter $i$ determined based on foreground features and a variance of the background 146. A simple example for a threshold model 162 includes, e.g., $\delta_i > 3\,\sigma_b$.

Optionally, the threshold values 160 determined according to the threshold models 162 are scaled by spot-specific weighting factors 164, e.g., by element-wise multiplication. Alternatively or in addition, the threshold values 160 are shifted by spot-specific offset values 166, e.g., by element-wise addition.

A binary result of the comparison 226, e.g., whether or not the threshold value is exceeded by the spot $m$ at time $n$, is represented by a binary matrix 168. The binary matrix 168 is input to a decision tree 170 in the step 230, which provides the result of the immunoassay.

Optionally, a subset of the spots 112 (e.g., the spots $m_i$, $i=1..s$) are configured with varying sensitivity to the same analyte 102, e.g. by including different concentrations of the binding molecule in the spots $m_i$, $i=1..s$. The parameters 150 of those spots $m_i$, $i=1..s$ may be fitted to a stored sensitivity curve (e.g., relative intensity as a function of concentration) to determine the concentration of the analyte 102.

Alternatively or in addition, the parameters 150 of the spots $m_i$ $i=1..s$ determine a mapping $S[i]$ according to

$$F[n,m_i] = S[m_i] \odot F[n,m_i].$$

The mapping $S[i]$ may be represented by a polynomial (e.g., of lower order than size $s$ of the subset) in the threshold concentration of the spot $m_i$. The polynomial is inverted to determine the analyte concentration.

Many advantages of the present invention will be fully understood from the foregoing description, and it will be apparent that various changes may be made in the form, construction and arrangement of the units and devices without departing from the scope of the invention and/or without sacrificing all of its advantages. Since the invention can be varied in many ways, it will be recognized that the invention should be limited only by the scope of the following claims.

## Claims

1. A device (100) for performing an immunoassay of analytes (102) in a sample (104), the device comprising:

a membrane (106) including a first site (108), a second site (110) laterally separated from the first site and multiple spots (112) arranged between the first site (108) and the second site (110), each spot (112) including binding molecules for selectively binding the analytes (102) at the corresponding spot (112);
a liquid phase (114) configured to carry the sample (104) and labels (116) for optically signaling the presence of the analytes (102), and to laterally flow (118) from the first site (108) across the spots (112) to the second site (110);
an image acquisition unit (128) configured to acquire (202) a sequence (142) of images (134), wherein each image captures the multiple spots (112), and the sequence of images is indicative of optical signals from the labels (116) at different points in time (154) during the lateral flow (118); and
a controller (130) coupled to the image acquisition unit (128) and configured to analyze (204) the sequence of images during the lateral flow (118) by extracting signal features of each of the multiple spots (112) for each of the points in time (154) of the sequence, determining parameters (150) of at least one signal model (152) for each of the multiple spots (112) based on the extracted signal features of the corresponding spot (112) and comparing the parameters with one or more threshold values,

(i) wherein a hydrodynamic correlation between signals from different spots (112) is compensated using the temporal sequence of images, the hydrodynamic correlation comprising spots (112) in the direction of the lateral flow (118) downstream of other spots (112) receiving less analytes than the other spots (112), the controller (130) assigning correspondingly lower threshold values to downstream spots (112) to compensate such a geometrically caused gradient in the concentration of one or more of the analytes, and/or
(ii) wherein the controller (130) is further configured to scale the threshold values by weighting factors assigned to each of the spots (112), wherein different weighting factors are assigned to compensate different flow rates or different velocities of the liquid phase (114).

**2.** The device of claim 1, wherein the controller (130) is further configured to output (206) a result of the immunoassay based on the sequence of images during the lateral flow (118).

**3.** The device of claim 2, wherein the controller (130) is configured to segment each image (134) of the sequence into subimages (136), optionally wherein each subimage (136) includes one of the spots (112).

**4.** The device of claim 2 or 3, wherein the controller (130) is configured to at least one of
retrieve coordinates for the multiple spots (112) or subimages (136) within the images (134) from memory at the device; and
determine coordinates for the multiple spots (112) or subimages (136) within the images (134) by processing at least one of the images.

**5.** The device of claim 3 or 4, wherein each of the subimages includes a foreground (144) and a background (146) of the corresponding one spot.

**6.** The device of claim 5, wherein the controller (130) is configured to separate (218) the foreground (144) and the background (146), optionally based on at least one of intensity, color, contrast, contour and texture.

**7.** The device of any one of claims 2 to 6, wherein the controller is configured to extract (212; 220, 222) signal features (140) of each of the multiple spots (112) for each of the points in time (154) of the sequence (142).

**8.** The device of claim 7, wherein the signal features (140) include at least one of a mean intensity of the spot (112), a variance of the intensity at the spot (112), a contrast of the spot (112), a texture of the spot (112), a contour of the spot (112) and an area size of the spot (112).

**9.** The device of claim 7 or 8, wherein the controller (130) is configured to determine parameters (150) of at least one signal model (152) for each of the multiple spots (112) based on the extracted signal features of the corresponding spot (112).

**10.** The device of claim 9, wherein each signal model (152) includes a dependency on the points in time of the sequence, and the controller (130) determines the parameters (150) of each spot (112) based on the signal features (140) of the corresponding spot (112) for the points in time (154) of the sequence (142) acquired up until real-time during the lateral flow (118).

**11.** The device of claim 10, wherein the controller (130) indicates completion of at least one of the acquisition (202) and the analysis (204) depending on a goodness of the determined parameters (150).

**12.** The device of any one of claims 9 to 11, wherein the controller (130) is configured to compare (226) the parameters (150) with one or more threshold values (160) and to output (206) the result of the immunoassay based on the comparison (226).

**13.** The device of claim 12, wherein the controller (130) is configured to generate a binary matrix (168) that is indicative of the comparison for each of the multiple spots (112) and for each of the points in time (154).

**14.** The device of claim 13, wherein the binary matrix (168) is input (230) to a binary decision tree (170) and the result of the immunoassay is based on an output of the binary decision tree (170).

**15.** The device of any one of claims 12 to 14, wherein the controller (130) computes (228) the threshold values (160) according to a threshold model (162) depending on at least one of the parameters (150).

**16.** The device of any one of claims 1 to 15, wherein the spots (112) are arranged in an array including two or more rows of spots (112), each row extending between the first site (108) and the second site (110).

**17.** The device of claim 16 in conjunction with any one of claims 12 to 15, wherein the threshold values (160) are at least one of shifted by offsets (166) values assigned to each of the spots (112) and scaled by weighting factors (164) assigned to each of the spots (112).

**18.** The device of claim 17, wherein at least one of the offsets (166) values and the weighting factors (164) in at least one of the rows decrease from the spot (112) closest to the first site to the spot (112) closest to the second site.

**19.** The device of any one of claims 1 to 18, wherein the multiple spots (112) are different in at least one of a concentration of the binding molecules and a selectivity of the binding molecules.

**20.** The device of claim 19 in conjunction with claim 9, wherein the controller (130) is configured to determine a concentration of at least one of the analytes (102) by fitting the parameters (150) of spots (112) including different concentrations of the same binding molecules selective to the analyte to a sensitivity curve of said binding molecules.

**21.** A method of performing an immunoassay of analytes (102) in a sample (104), the method comprising:

providing a membrane (106) including a first site (108), a second site (110) laterally separated from the first site and multiple spots (112) arranged between the first site and the second site, each spot (112) including binding molecules for selectively binding the analytes at the corresponding spot (112);

providing a liquid phase (114) configured to carry the sample (104) and labels (116) for optically signaling the presence of the analytes, and to laterally flow (118) from the first site (108) across the spots (112) to the second site (110);

acquiring (202) a sequence (136) of images (134), wherein each image captures the multiple spots (112), and the sequence (142) of images (134) is indicative of optical signals from the labels (116) at different points in time (154) during the lateral flow (118); and

analyzing (204) the sequence of images during the lateral flow (118) by extracting signal features of each of the multiple spots (112) for each of the points in time (154) of the sequence, determining parameters (150) of at least one signal model (152) for each of the multiple spots (112) based on the extracted signal features of the corresponding spot (112) and comparing the parameters with one or more threshold values,

(i) wherein a hydrodynamic correlation between signals from different spots (112) is compensated using the temporal sequence of images, the hydrodynamic correlation comprising spots (112) in the direction of the lateral flow (118) downstream of other spots (112) receiving less analytes than the other spots (112), correspondingly lower threshold values being assigned to downstream spots (112) to compensate such a geometrically caused gradient in the concentration of one or more of the analytes, and/or

(ii) wherein the threshold values are scaled by weighting factors assigned to each of the spots (112), wherein different weighting factors are assigned to compensate different flow rates or different velocities of the liquid phase (114).

**Patentansprüche**

1. Vorrichtung (100) zur Durchführung eines Immunoassays von Analyten (102) in einer Probe (104), wobei die Vorrichtung umfasst:

eine Membran (106) mit einer ersten Stelle (108), einer zweiten Stelle (110), die seitlich von der ersten Stelle getrennt ist, und mehreren Spots (112), die zwischen der ersten Stelle (108) und der zweiten Stelle (110) angeordnet sind, wobei jeder Spot (112) Bindungsmoleküle zur selektiven Bindung der Analyten (102) an den entsprechenden Spot (112) enthält;

eine flüssige Phase (114), die so konfiguriert ist, dass sie die Probe (104) und Markierungen (116) zum optischen Signalisieren der Anwesenheit der Analyten (102) trägt und seitlich von der ersten Stelle (108) über die Spots (112) zur zweiten Stelle (110) fließt (118);

eine Bilderfassungseinheit (128), die so konfiguriert ist, dass sie eine Sequenz (142) von Bildern (134) erfasst (202), wobei jedes Bild die mehreren Punkte (112) erfasst und die Sequenz von Bildern optische Signale von den Etiketten (116) zu verschiedenen Zeitpunkten (154) während des lateralen Flusses (118) anzeigt; und

einen Controller (130), der mit der Bilderfassungseinheit (128) gekoppelt und so konfiguriert ist, dass er die Bildsequenz während des lateralen Flusses (118) analysiert (204), indem er Signalmerkmale von jedem der mehreren Punkte (112) für jeden der Zeitpunkte (154) der Sequenz extrahiert, Parameter (150) von mindestens einem Signalmodell (152) für jeden der mehreren Punkte (112) auf der Grundlage der extrahierten Signalmerkmale des entsprechenden Punktes (112) bestimmt und die Parameter mit einem oder mehreren Schwellenwerten vergleicht,

(i) wobei eine hydrodynamische Korrelation zwischen Signalen von verschiedenen Spots (112) unter Verwendung der zeitlichen Abfolge von Bildern kompensiert wird, wobei die hydrodynamische Korrelation Spots (112) in der Richtung der lateralen Strömung (118) stromabwärts von anderen Spots (112) umfasst, die weniger Analyten als die anderen Spots (112) empfangen, wobei der Controller (130) stromabwärts gelegenen Spots (112) entsprechend niedrigere Schwellenwerte zuweist, um einen solchen geometrisch bedingten Gradienten in der Konzentration eines oder mehrerer der Analyten zu kompensieren, und/oder

(ii) wobei der Controller (130) ferner so konfiguriert ist, dass er die Schwellenwerte durch Gewichtungsfaktoren skaliert, die jedem der Punkte (112) zugeordnet sind, wobei unterschiedliche Gewichtungsfaktoren zugeordnet sind, um unterschiedliche Durchflussraten oder unterschiedliche Geschwindigkeiten der flüssigen Phase (114) zu kompensieren.

2. Vorrichtung nach Anspruch 1, wobei der Controller (130) ferner so konfiguriert ist, dass er ein Ergebnis des Immunoassays auf der Grundlage der Sequenz von Bildern während des lateralen Flusses ausgibt (206).

**3.** Vorrichtung nach Anspruch 2, wobei der Controller (130) so konfiguriert ist, dass er jedes Bild (134) der Sequenz in Teilbilder (136) segmentiert, wobei jedes Teilbild (136) optional einen der Spots (112) enthält.

**4.** Vorrichtung nach Anspruch 2 oder 3, wobei der Controller (130) für mindestens eines der folgenden konfiguriert ist

Abrufen von Koordinaten für die mehreren Spots (112) oder Unterbilder (136) innerhalb der Bilder (134) aus dem Speicher des Geräts; und Bestimmen von Koordinaten für die mehreren Spots (112) oder Unterbilder (136) innerhalb der Bilder (134), indem mindestens eines der Bilder bearbeitet wird.

**5.** Vorrichtung nach Anspruch 3 oder 4, wobei jedes der Unterbilder einen Vordergrund (144) und einen Hintergrund (146) des entsprechenden einen Spots enthält.

**6.** Vorrichtung nach Anspruch 5, wobei der Controller (130) so konfiguriert ist, dass er den Vordergrund (144) und den Hintergrund (146) trennt (218), wahlweise auf der Basis von Intensität, Farbe, Kontrast, Kontur oder Textur.

**7.** Vorrichtung nach einem der Ansprüche 2 bis 6, wobei der Controller so konfiguriert ist, dass er Signalmerkmale (140) von jedem der mehreren Punkte (112) für jeden der Zeitpunkte (154) der Sequenz (142) extrahiert (212; 220, 222).

**8.** Vorrichtung nach Anspruch 7, wobei die Signalmerkmale (140) mindestens eines von einer mittleren Intensität des Spots (112), einer Varianz der Intensität an dem Spot (112), einem Kontrast des Spots (112), einer Textur des Spots (112), einer Kontur des Spots (112) und einer Flächengröße des Spots (112) umfassen.

**9.** Vorrichtung nach Anspruch 7 oder 8, wobei der Controller (130) so konfiguriert ist, dass er Parameter (150) mindestens eines Signalmodells (152) für jeden der mehreren Spots (112) auf der Grundlage der extrahierten Signalmerkmale des entsprechenden Spots (112) bestimmt.

**10.** Vorrichtung nach Anspruch 9, wobei jedes Signalmodell (152) eine Abhängigkeit von den Zeitpunkten der Sequenz enthält und der Controller (130) die Parameter (150) jedes Punkts (112) auf der Grundlage der Signalmerkmale (140) des entsprechenden Punkts (112) für die Zeitpunkte (154) der Sequenz (142) bestimmt, die bis zur Echtzeit während des lateralen Flusses (118) erfasst wurden.

**11.** Vorrichtung nach Anspruch 10, bei der der Controller (130) den Abschluss der Erfassung (202) und/oder der Analyse (204) in Abhängigkeit von einer Güte der ermittelten Parameter (150) anzeigt.

**12.** Vorrichtung nach einem der Ansprüche 9 bis 11, wobei der Controller (130) so konfiguriert ist, dass er die Parameter (150) mit einem oder mehreren Schwellenwerten (160) vergleicht (226) und das Ergebnis des Immunoassays auf der Grundlage des Vergleichs (226) ausgibt (206).

**13.** Vorrichtung nach Anspruch 12, wobei der Controller (130) so konfiguriert ist, dass er eine binäre Matrix (168) erzeugt, die den Vergleich für jeden der mehreren Spots (112) und für jeden der Zeitpunkte (154) anzeigt.

**14.** Vorrichtung nach Anspruch 13, wobei die binäre Matrix (168) in einen binären Entscheidungsbaum (170) eingegeben (230) wird und das Ergebnis des Immunoassays auf einer Ausgabe des binären Entscheidungsbaums (170) basiert.

**15.** Vorrichtung nach einem der Ansprüche 12 bis 14, wobei der Controller (130) die Schwellenwerte (160) gemäß einem Schwellenwertmodell (162) in Abhängigkeit von mindestens einem der Parameter (150) berechnet (228).

**16.** Vorrichtung nach einem der Ansprüche 1 bis 15, wobei die Spots (112) in einer Anordnung mit zwei oder mehr Reihen von Spots (112) angeordnet sind, wobei sich jede Reihe zwischen der ersten Stelle (108) und der zweiten Stelle (110) erstreckt.

**17.** Vorrichtung des Anspruchs 16 in Verbindung mit einem der Ansprüche 12 bis 15, wobei die Schwellenwerte (160) mindestens einer der Werte durch Versätze (166), die jedem der Punkte (112) zugeordnet sind, verschoben und durch Gewichtungsfaktoren (164), die jedem der Punkte (112) zugeordnet sind, skaliert sind.

**18.** Vorrichtung nach Anspruch 17, wobei mindestens einer der Werte der Offsets (166) und der Gewichtungsfaktoren (164) in mindestens einer der Reihen von dem Punkt (112), der der ersten Stelle am nächsten liegt, zu dem Punkt (112), der der zweiten Stelle am nächsten liegt, abnimmt.

**19.** Vorrichtung nach einem der Ansprüche 1 bis 18, wobei sich die Mehrfachspots (112) in mindestens einer Konzentration der Bindungsmoleküle und einer Selektivität der Bindungsmoleküle unterscheiden.

20. Vorrichtung nach Anspruch 19 in Verbindung mit Anspruch 9, wobei der Controller (130) so konfiguriert ist, dass er eine Konzentration von mindestens einem der Analyten (102) bestimmt, indem er die Parameter (150) der Spots (112), einschließlich unterschiedlicher Konzentrationen derselben Bindungsmoleküle, die für den Analyten selektiv sind, an eine Empfindlichkeitskurve der genannten Bindungsmoleküle anpasst.

21. Verfahren zur Durchführung eines Immunoassays von Analyten (102) in einer Probe (104), wobei das Verfahren umfasst:

Bereitstellen einer Membran (106) mit einer ersten Stelle (108), einer zweiten Stelle (110), die seitlich von der ersten Stelle getrennt ist, und mehreren Spots (112), die zwischen der ersten Stelle und der zweiten Stelle angeordnet sind, wobei jeder Spot (112) Bindungsmoleküle zur selektiven Bindung der Analyten an den entsprechenden Spot (112) enthält;

Bereitstellen einer flüssigen Phase (114), die so konfiguriert ist, dass sie die Probe (104) und Markierungen (116) zum optischen Signalisieren der Anwesenheit der Analyten trägt und seitlich von der ersten Stelle (108) über die Spots (112) zur zweiten Stelle (110) fließt (118);

Erfassen (202) einer Sequenz (136) von Bildern (134), wobei jedes Bild die mehreren Punkte (112) erfasst und die Sequenz (142) von Bildern (134) optische Signale von den Etiketten (116) zu verschiedenen Zeitpunkten (154) während des lateralen Flusses (118) anzeigt; und

Analysieren (204) der Bildsequenz während des lateralen Flusses (118) durch Extrahieren von Signalmerkmalen jedes der mehreren Spots (112) für jeden der Zeitpunkte (154) der Sequenz, Bestimmen von Parametern (150) mindestens eines Signalmodells (152) für jeden der mehreren Spots (112) auf der Grundlage der extrahierten Signalmerkmale des entsprechenden Spots (112) und Vergleichen der Parameter mit einem oder mehreren Schwellenwerten,

(i) wobei eine hydrodynamische Korrelation zwischen Signalen von verschiedenen Spots (112) unter Verwendung der zeitlichen Abfolge von Bildern kompensiert wird, wobei die hydrodynamische Korrelation Spots (112) in der Richtung der lateralen Strömung (118) stromabwärts von anderen Spots (112) umfasst, die weniger Analyten als die anderen Spots (112) empfangen, wobei entsprechend niedrigere Schwellenwerte stromabwärts liegenden Spots (112) zugeordnet werden, um einen solchen geometrisch bedingten Gradienten in der Konzentration eines oder mehrerer der Analyten zu kompensieren, und/oder

(ii) wobei die Schwellenwerte durch Wichtungsfaktoren skaliert werden, die jedem der Spots (112) zugeordnet sind, wobei unterschiedliche Wichtungsfaktoren zugeordnet werden, um unterschiedliche Flussraten oder unterschiedliche Geschwindigkeiten der flüssigen Phase (114) zu kompensieren.

## Revendications

1. Dispositif (100) pour effectuer un immunoessai d'analytes (102) dans un échantillon (104), le dispositif comprenant :

une membrane (106) comportant un premier site (108), un second site (110) séparé latéralement du premier site et de multiples points (112) agencés entre le premier site (108) et le second site (110), chaque point (112) comportant des molécules de liaison pour lier sélectivement les analytes (102) au point (112) correspondant ;

une phase liquide (114) configurée pour porter l'échantillon (104) et des marqueurs (116) pour signaler optiquement la présence des analytes (102), et pour s'écouler latéralement (118) depuis le premier site (108) à travers les points (112) vers le second site (110) ;

une unité d'acquisition d'image (128) configurée pour acquérir (202) une séquence (142) d'images (134), dans lequel chaque image capture les multiples points (112), et la séquence d'images indique des signaux optiques provenant des marqueurs (116) à différents moments dans le temps (154) pendant l'écoulement latéral (118) ; et

un dispositif de commande (130) couplé à l'unité d'acquisition d'image (128) et configuré pour analyser (204) la séquence d'images pendant l'écoulement latéral (118) par l'extraction de caractéristiques de signal de chacun des multiples points (112) pour chacun des moments dans le temps (154) de la séquence, la détermination de paramètres (150) d'au moins un modèle de signal (152) pour chacun des multiples points (112) sur la base des caractéristiques de signal extraites du point (112) correspondant et la comparaison des paramètres avec une ou plusieurs valeurs seuils,

(i) dans lequel une corrélation hydrodynamique entre des signaux provenant de différents points (112) est compensée en utilisant la séquence temporelle d'images, la corrélation hydrodynamique comprenant des points (112) dans la direction de l'écoulement latéral (118) en aval d'autres points (112) recevant moins d'analytes que les autres points (112), le dispositif de commande (130) attribuant des valeurs seuils proportionnellement inférieures aux points (112) en aval pour compenser un tel gradient dû à la géométrie dans la concentration d'un ou de plusieurs des analytes, et/ou (ii) dans lequel le dispositif de commande (130) est configuré en outre pour mettre à l'échelle les valeurs seuils par des facteurs de pondération attribués à chacun des points (112), dans lequel différents facteurs de pondération sont attribués pour compenser différents débits ou différentes vitesses de la phase liquide (114).

2. Dispositif selon la revendication 1, dans lequel le dispositif de commande (130) est configuré en outre pour sortir (206) un résultat de l'immunoessai sur la base de la séquence d'images pendant l'écoulement latéral (118).

3. Dispositif selon la revendication 2, dans lequel le dispositif de commande (130) est configuré pour segmenter chaque image (134) de la séquence en sous-images (136), facultativement dans lequel chaque sous-image (136) comporte l'un des points (112).

4. Dispositif selon la revendication 2 ou 3, dans lequel le dispositif de commande (130) est configuré pour au moins l'un parmi
récupérer des coordonnées pour les multiples points (112) ou sous-images (136) dans les images (134) à partir d'une mémoire sur le dispositif ; et
déterminer des coordonnées pour les multiples points (112) ou sous-images (136) dans les images (134) par le traitement d'au moins l'une des images.

5. Dispositif selon la revendication 3 ou 4, dans lequel chacune des sous-images comporte un premier plan (144) et un arrière-plan (146) du point correspondant.

6. Dispositif selon la revendication 5, dans lequel le dispositif de commande (130) est configuré pour séparer (218) le premier plan (144) et l'arrière-plan (146), facultativement sur la base d'au moins l'un parmi une intensité, une couleur, un contraste, un contour et une texture.

7. Dispositif selon l'une quelconque des revendications 2 à 6, dans lequel le dispositif de commande est configuré pour extraire (212 ; 220, 222) des caractéristiques de signal (140) de chacun des multiples points (112) pour chacun des moments dans le temps (154) de la séquence (142).

8. Dispositif selon la revendication 7, dans lequel les caractéristiques de signal (140) comportent au moins l'un parmi une intensité moyenne du point (112), une variance de l'intensité au niveau du point (112), un contraste du point (112), une texture du point (112), un contour du point (112) et une taille de zone du point (112).

9. Dispositif selon la revendication 7 ou 8, dans lequel le dispositif de commande (130) est configuré pour déterminer les paramètres (150) d'au moins un modèle de signal (152) pour chacun des multiples points (112) sur la base des caractéristiques de signal extraites du point (112) correspondant.

10. Dispositif selon la revendication 9, dans lequel chaque modèle de signal (152) comporte une dépendance aux moments dans le temps de la séquence, et le dispositif de commande (130) détermine les paramètres (150) de chaque point (112) sur la base des caractéristiques du signal (140) du point (112) correspondant pour les moments dans le temps (154) de la séquence (142) acquis jusqu'au temps réel pendant de l'écoulement latéral (118).

11. Dispositif selon la revendication 10, dans lequel le dispositif de commande (130) indique la fin d'au moins l'une parmi l'acquisition (202) et l'analyse (204) en fonction de la qualité des paramètres (150) déterminés.

12. Dispositif selon l'une quelconque des revendications 9 à 11, dans lequel le dispositif de commande (130) est configuré pour comparer (226) les paramètres (150) avec une ou plusieurs valeurs seuils (160) et pour sortir (206) le résultat de l'immunoessai sur la base de la comparaison (226).

13. Dispositif selon la revendication 12, dans lequel le dispositif de commande (130) est configuré pour générer une matrice binaire (168) qui indique la comparaison pour chacun des multiples points (112) et pour chacun des moments dans le temps (154).

14. Dispositif selon la revendication 13, dans lequel la matrice binaire (168) est entrée (230) dans un arbre de décision binaire (170) et le résultat de l'immunoessai est basé sur une sortie de l'arbre de décision binaire (170).

15. Dispositif selon l'une quelconque des revendications 12 à 14, dans lequel le dispositif de commande (130) calcule (228) les valeurs seuils (160) selon un modèle de seuil (162) en fonction d'au moins l'un des paramètres (150).

**16.** Dispositif selon l'une quelconque des revendications 1 à 15, dans lequel les points (112) sont agencés en un réseau comportant deux ou plus de deux rangées de points (112), chaque rangée s'étendant entre le premier site (108) et le second site (110).

**17.** Dispositif selon la revendication 16 conjointement avec l'une quelconque des revendications 12 à 15, dans lequel les valeurs seuils (160) sont au moins l'une parmi décalées par des valeurs de décalages (166) attribuées à chacun des points (112) et mises à l'échelle par des facteurs de pondération (164) attribués à chacun des points (112).

**18.** Dispositif selon la revendication 17, dans lequel au moins l'un parmi les valeurs de décalage (166) et les facteurs de pondération (164) dans au moins l'une des rangées diminuent à partir du point (112) le plus proche du premier site vers le point (112) le plus proche du second site.

**19.** Dispositif selon l'une quelconque des revendications 1 à 18, dans lequel les multiples points (112) sont différents en ce qui concerne au moins l'une parmi une concentration des molécules de liaison et une sélectivité des molécules de liaison.

**20.** Dispositif selon la revendication 19 conjointement avec la revendication 9, dans lequel le dispositif de commande (130) est configuré pour déterminer une concentration d'au moins l'un des analytes (102) par l'ajustement des paramètres (150) des points (112) comportant différentes concentrations des mêmes molécules de liaison sélectives de l'analyte à une courbe de sensibilité desdites molécules de liaison.

**21.** Procédé de mise en œuvre d'un immunoessai d'analytes (102) dans un échantillon (104), le procédé comprenant :

la fourniture d'une membrane (106) comportant un premier site (108), un second site (110) séparé latéralement du premier site et de multiples points (112) agencés entre le premier site et le second site, chaque point (112) comportant des molécules de liaison pour lier sélectivement les analytes au point (112) correspondant ;
la fourniture une phase liquide (114) configurée pour porter l'échantillon (104) et des marqueurs (116) pour signaler optiquement la présence des analytes, et pour s'écouler latéralement (118) depuis le premier site (108) à travers les points (112) vers le second site (110) ;
l'acquisition (202) d'une séquence (136) d'image (134), dans lequel chaque image capture les multiples points (112), et la séquence (142) d'images (134) indique des signaux optiques provenant des marqueurs (116) à différents moments dans le temps (154) pendant l'écoulement latéral (118) ; et
l'analyse (204) de la séquence d'image pendant l'écoulement latéral (118) par l'extraction de caractéristiques de signal de chacun des multiples points (112) pour chacun des moments dans le temps (154) de la séquence, la détermination de paramètres (150) d'au moins un modèle de signal (152) pour chacun des multiples points (112) sur la base des caractéristiques de signal extraites du point (112) correspondant et la comparaison des paramètres avec une ou plusieurs valeurs seuils,

(i) dans lequel une corrélation hydrodynamique entre des signaux provenant de différents points (112) est compensée en utilisant la séquence temporelle d'images, la corrélation hydrodynamique comprenant des points (112) dans la direction de l'écoulement latéral (118) en aval d'autres points (112) recevant moins d'analytes que les autres points (112), des valeurs seuils proportionnellement inférieures étant attribuées aux points (112) en aval pour compenser un tel gradient dû à la géométrie dans la concentration d'un ou de plusieurs des analytes, et/ou
(ii) dans lequel les valeurs seuils sont mises à l'échelle par des facteurs de pondération attribués à chacun des points (112), dans lequel différents facteurs de pondération sont attribués pour compenser différents débits ou différentes vitesses de la phase liquide (114).

FIG. 1

EP 3 279 662 B1

EP 3 279 662 B1

FIG. 2

FIG. 3

$I_{\mathrm{ROI}}[n,m]$ ⬚ —136

Enhance ROI $I_{\mathrm{ROI}}[n,m]$ —216

Separate ROI —218

Background                    Foreground

146                          144

Extract background features      Extract foreground features

222                          220

$\underline{f}_{\mathrm{fg}}[n,m]$              $\underline{f}_{\mathrm{bg}}[n,m]$

$$\underline{f}[n,m] = \begin{pmatrix} \underline{f}_{\mathrm{fg}}[n,m] \\ \underline{f}_{\mathrm{bg}}[n,m] \end{pmatrix}$$

140

FIG. 4

EP 3 279 662 B1

224

Signal model — 152

140

$\underline{f}[n,m]$ → Determine model parameters

150

$$\mathbf{F}[N,m] = \begin{pmatrix} \mu_{\mu_f} & \sigma_{\mu_f} & t_{\mu_f} & \cdots \\ \mu_{\sigma_f} & \sigma_{\sigma_f} & t_{\sigma_f} & \cdots \\ \vdots & \vdots & \vdots & \ddots \end{pmatrix}$$

FIG. 5

$f_i^{model}[n,m]$ — 152

$f_i[n,m]$

140

156

$m$
(Spots)

$n$ (Time) — 154

$N$
(Real-time)

FIG. 6

$\mathbf{F}[n,m]$ —2— 150

228

162 —2— | Threshold model | → | Compute threshold |

160 —2— $T[\text{n},\text{m}]$

Compare with threshold values —2— 226

164 —2— $G[m]$ → ○

150

166 —2— $\Delta[m]$ → +

—160

$\mathbf{R}[\text{n},\text{m}] = \begin{pmatrix} 1 & 0 & 1 & \cdots \\ 0 & 1 & 1 & \cdots \\ \vdots & \vdots & \vdots & \ddots \end{pmatrix}$ —2—168

170 —2→

Logically combine result of comparison —2— 230

*True*　　*False*

Output result —2— 206

FIG. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **D. J. CARTER ; R. B. CARY.** Lateral flow microarrays: a novel platform for rapid nucleic acid detection based on miniaturized lateral flow chromatography. *Nucleic Acids Research,* 2007, vol. 32 (10), e74